Europäisches Patentamt

European Patent Office     (11) Veröffentlichungsnummer: **0 004 656**
                                                        **B1**
Office européen des brevets

(12) **EUROPÄISCHE PATENTSCHRIFT**

(19)

(45) Veröffentlichungstag der Patentschrift:     (51) Int. Cl.³: **C 07 C 27/06, C 07 C 31/08,**
   **07.10.81**                                          **C 07 C 47/06, C 07 C 53/08**

(21) Anmeldenummer: **79100995.4**

(22) Anmeldetag: **31.03.79**

(54) Verfahren zur Herstellung sauerstoffhaltiger Kohlenstoffverbindungen aus Synthesegas.

(30) Priorität: 04.04.78 **DE 2814427**         (73) Patentinhaber: **HOECHST AKTIENGESELLSCHAFT,**
            10.06.78 **DE 2825598**                  **Postfach 80 03 20, D-6230 Frankfurt/Main 80 (DE)**
            18.11.78 **DE 2850201**

                                             (72) Erfinder: **Wunder, Friedrich, Dr., Jahnstrasse 46,**
(43) Veröffentlichungstag der Anmeldung:              **D-6093 Flörsheim am Main (DE)**
   **17.10.79 Patentblatt 79/21**                   Erfinder: **Arpe, Hans-Jürgen, Dr., Am Hirtengraben 19,**
                                                    **D-6233 Kelkheim (Taunus) (DE)**
                                                    Erfinder: **Leupold, Ernst Ingo, Dr., Am Zäunefeld 15,**
(45) Bekanntmachung des Hinweises auf die Patenterteilung:   **D-6392 Neu-Anspach (DE)**
   **07.10.81 Patentblatt 81/40**                   Erfinder: **Schmidt, Hans-Joachim, Dr., Burgenblick 6,**
                                                    **D-6240 Königstein/Taunus (DE)**

(84) Benannte Vertragsstaaten:
   **BE DE FR GB IT NL**

(56) Entgegenhaltungen:
   **FR-A-2 234 256**
   **FR-A-2 234 257**
   **FR-A-2 277 804**
   **FR-A-2 317 260**

Verfahren zur Herstellung sauerstoffhaltiger Kohlenstoffverbindungen aus Synthesegas

Die Erfindung betrifft ein Verfahren zur Herstellung sauerstoffhaltiger Verbindungen mit vorzugsweise zwei Kohlenstoffatomen im Molekül und insbesondere die Herstellung von Essigsäure, Ethanol, Acetaldehyd und gegebenenfalls ihren Folgeprodukten durch Umsetzung von Synthesegas, dh. Kohlenmonoxid und Wasserstoff, an Rhodiummetall enthaltenden Trägerkatalysatoren.

Aus FR-A-2 234 257 ist die Herstellung von Ethylenglykol und Methanol aus Synthesegas an Katalysatoren bekannt, die Metallcarbonyle, vorzugsweise Rhodiumcarbonyle, enthalten. Analog werden gemäss FR-A-2 277 804 und FR-A-2 234 256 Polyalkohole und ihre Derivate an Rhodiumcarbonyl-Katalysatoren hergestellt.

In FR-A-2 317 260 wird die Herstellung von Essigsäure, Ethanol und Acetaldehyd aus Synthesegas an geträgerten Rhodiummetall-Katalysatoren beschrieben. Als Träger wird unter anderem Magnesiumoxid genannt, das aber in Wirklichkeit nicht geeignet sein dürfte, da es gegen die bei dem Verfahren gebildete Essigsäure nicht beständig ist.

Aus den deutschen Auslegeschriften 2 503 233 und 2 503 204 ist ebenfalls bereits bekannt, dass die Gasphasenumsetzung von Synthesegas an Rhodiummetall enthaltenden Katalysatoren im wesentlichen zu Gemischen sauerstoffhaltiger Produkte mit zwei Kohlenstoffatomen im Molekül, wie Essigsäure, Ethanol und/oder Acetaldehyd, führen kann. Die Selektivität zu den einzelnen Verbindungen hängt von den Reaktionsbedingungen ab und kann durch Zusatz von Eisensalzen zugunsten von Ethanol beeinflusst werden.

Aus der deutschen Offenlegungsschrift 2 628 463 ist weiter bekannt, dass durch Zusatz von Mangan zu Rhodium enthaltenden Katalysatoren deren Aktivität verbessert (bzw. zur Erzielung gleicher Aktivität die Rhodiummenge verringert) werden kann, ohne dass die Selektivität zu den sauerstoffhaltigen Verbindungen entscheidend verändert wird. Dies hat aber zur Folge, dass mit erhöhter Aktivität der Katalysatoren auch eine erhöhte Menge an Nebenprodukten anfällt, was zusätzliche Aufwendungen zu ihrer Abtrennung und gegebenenfalls ihrer Verwertung erforderlich macht. So geht beispielsweise aus der Tabelle der deutschen Offenlegungsschrift 2 628 463 hervor, dass die aktivsten der dort genannten Katalysatoren Raumzeitausbeuten von mehr als 350 g (bis über 400 g) sauerstoffhaltige $C_2$-Produkte pro Liter Katalysator und Stunde erreichen, dass aber die Gesamtselektivität zu diesen $C_2$-Produkten 60%, bezogen auf umgesetztes Kohlenmonoxid, nicht übersteigt. 40% und mehr des umgesetzten Kohlenmonoxids reagieren zu anderen Produkten, beispielsweise zu Kohlenwasserstoffen.

Somit ergibt sich als Aufgabe, Rhodiummetall enthaltende Katalysatoren zur Umsetzung von Synthesegas in ihrer Selektivität zu sauerstoffhaltigen $C_2$-Produkten zu verbessern, und damit die Wirtschaftlichkeit eines solchen Verfahrens für diese technisch bedeutsamen Zwischenprodukte zu erhöhen.

Es wurde nun gefunden, dass Aktivität und/oder Selektivität der Rhodiummetall-Katalysatoren verbessert werden können, wenn sie als Cokatalysatoren Magnesiumsalze oder -komplexverbindungen oder Verbindungen des Magnesiums mit Oxyden von Elementen der Gruppen III bis VI des periodischen Systems, sowie Halogenidionen enthalten.

Gegenstand der Erfindung ist daher ein Verfahren zur Herstellung von Essigsäure, Ethanol, Acetaldehyd und gegebenenfalls ihren Folgeprodukten durch Umsetzung von Kohlenmonoxid mit Wasserstoff an Rhodiummetall enthaltenden Trägerkatalysatoren, das dadurch gekennzeichnet ist, dass die Katalysatoren zusätzlich Halogenidionen und Magnesiumsalze und/oder -komplexverbindungen und/oder Verbindungen des Magnesiums mit Oxiden von Elementen der III. bis VI. Gruppe des periodischen Systems enthalten.

Damit liegt ein neuartiges cokatalytisch wirkendes System vor, das gegenüber den bisher bekannt gewordenen Cokatalysatoren (Mangan und Eisen) eine verbesserte Gesamtselektivität zu den genannten sauerstoffhaltigen Verbindungen (Essigsäure, Ethanol, Acetaldehyd und gegebenenfalls ihre Folgeprodukte) aufweist. Überraschend dabei ist, dass anders als beim Mangan und Eisen, die in mehreren Wertigkeitsstufen vorkommen und deren katalytische Eigenschaften im allgemeinen auf ihrem Wertigkeitswechsel (Redox-Katalysatoren) beruhen, mit dem Magnesium ein Element zur Selektivitätssteuerung vorliegt, das nur in einer stabilen Wertigkeit (zweiwertig) vorkommt. Es ist also damit zu rechnen, dass die Funktion des Magnesiums zusammen mit den erfindungsgemäss notwendigen Halogenidionen im Mechanismus der Synthesegasumsetzung zu sauerstoffhaltigen $C_2$-Produkten eine grundlegend andere ist als beim Mangan oder Eisen. Es war zum weiteren nicht vorhersehbar, dass diese Wirkungsweise sich in solchem Mass auf die Erhöhung der Rhodium-Aktivität und -Selektivität auswirkt, dass die Wirtschaftlichkeit des vorliegenden Verfahrens wesentlich verbessert wird.

Sauerstoffhaltige Kohlenstoffverbindungen, die bei dem erfindungsgemässen Verfahren in hoher Selektivität entstehen, sind Essigsäure, Ethanol und/oder Acetaldehyd, ferner solche Produkte, die in einer Folgereaktion, z.B. durch Veresterung oder Kondensation, aus diesen Produkten unter den Reaktionsbedingungen gebildet werden können. Hierzu zählen z.B. Ethylacetat und das Diethylacetal des Acetaldehydes. Der Anteil an anderen sauerstoffhaltigen Verbindungen mit drei oder mehr Kohlenstoffatomen im Molekül ist sehr gering und liegt normalerweise unter 10 Mol %, bezogen auf umgesetztes Kohlenmonoxid. Die Gesamtselektivität zu sauerstoffhaltigen $C_2$-Verbindungen, einschliesslich der in Ethylacetat und Acetaldehyddiethylacetal umgewandelten Produkte, beträgt im allgemeinen über 75%, bezogen auf umgesetztes Kohlenmonoxid. Das restliche Kohlenmonoxid wird ausser zu den genannten sauerstoffhaltigen Produkten mit 3 und mehr Kohlenstoffatomen im wesentlichen zu Methan und anderen gasförmigen Kohlenwasserstoffen und in geringem Masse zu Kohlendioxid umgesetzt.

Geeignete Magnesiumverbindungen sind die einfachen anorganischen oder organischen Salze des Magnesiums, wie z.B. das Chlorid, Bromid, Nitrat, Formiat und Acetat. Ferner können das Oxid, das Hydroxid oder die Carbonate des Magnesiums verwandt werden, wenn man sie durch Behandlung mit Mineralsäure oder Carbonsäuren in die genannten Salze überführt. Geeignet sind ferner Komplexverbindungen des Magnesiums mit anorganischen oder organischen Liganden, wie z.B. Kaliummagnesiumtrichlorid, Magnesiumhexammindichlorid oder Magnesiumacetylacetonat. Besonders geeignet sind Verbindungen des Magnesiums mit Oxiden von Elementen der Gruppen III bis VI des periodischen Systems, beispielsweise natürliche oder synthetische Magnesiumaluminate, -aluminiumsilikate, -metasilikate, -orthosilikate, -titanate, -zirkonate und -chromite, wobei diese Verbindungen gleichzeitig auch als Trägersubstanz für das Rhodium verwendet werden können.

Man kann aber auch Magnesium mittels Ionenaustausch an Kationenaustauscher binden, die unter den Umsetzungsbedingungen beständig und auch als Träger für das Rhodium geeignet sind, beispielsweise die als Molsiebe bekannten natürlichen oder synthetischen Aluminiumsilikate.

Als Halogenide können die Chloride, Bromide oder Jodide von Metallen der I. bis VIII. Gruppe des periodischen Systems verwendet werden, da es nur auf das Anion ankommt. Besonders bevorzugt sind die Halogenide des Magnesiums oder Rhodiums, die auch beispielsweise durch Umsetzung von auf den Träger aufgebrachten Oxiden, Hydroxiden oder Carbonaten dieser beiden Elemente mit Halogenwasserstoff abspaltenden organischen Verbindungen (wie 1,1-Dichloräthan) auf dem Träger gebildet werden können.

Als Katalysatorträger können übliche Trägermaterialien mit unterschiedlichen spezifischen Oberflächen verwendet werden. Allerdings werden Träger mit spezifischen Oberflächen von 50 bis 1000 m²/g bevorzugt. Geeignet sind z.B. Kieselsäure, natürliche oder synthetische Silikate von Elementen der II. und VIII. Gruppe des periodischen Systems (also beispielsweise die Silikate des Magnesiums, Calciums, Aluminiums, der Seltenen Erden, des Titans, Zirkons, Mangans), ferner Aluminiumoxid, Zirkonoxid, Thoriumdioxid, Vanadinpentoxid, Zeolithe und Spinelle.

Zur Herstellung der Katalysatoren werden die Katalysatorträger mit den aktiven Komponenten gleichzeitig oder in aufeinanderfolgenden Stufen getränkt oder imprägniert und der Katalysator anschliessend reduziert. Bei Verwendung von Oxiden der Elemente der III. bis VI. Gruppe des periodischen Systems als Katalysatorträger besteht eine bevorzugte Herstellmethode der Katalysatoren darin, die Magnesiumverbindung und das Halogenid (die auch identisch sein können) auf den Träger aufzuziehen und den imprägnierten Träger durch Sintern bei höherer Temperatur ganz oder teilweise in ein Mischoxid des Magnesiums mit dem Element der III. bis VI. Gruppe, beispielsweise in das Magnesiumsilikat, überzuführen und anschliessend mit der Rhodiumverbindung zu imprägnieren.

Die Reduktion der eingesetzten Rhodiumverbindung zu metallischem Rhodium erfolgt durch Behandlung mit Reduktionsmitteln wie Wasserstoff oder Kohlenmonoxid oder deren Gemischen oder z.B. Methanol bei Temperaturen oberhalb 300°C, vorzugsweise bei Temperaturen zwischen 350 und 550°C. Im allgemeinen ist es zweckmässig, die Reduktion nicht mit den unverdünnten Reduktionsmitteln sondern mit einem zusätzlichen Anteil an Inertgasen, z.B. Stickstoff, Kohlendioxid oder auch Edelgasen vorzunehmen.

Die Konzentration an Rhodium, Magnesium und Halogeniden in den Katalysatoren kann in weiten Grenzen variiert werden; im allgemeinen liegen die Werte zwischen 0,1 und 20 Gew.-% für Rhodium, zwischen 0,1 und 25 Gew.-% für Magnesium und zwischen 0,1 und 20 Gew.-% für die Halogenidionen. Bevorzugt sind Katalysatoren mit 1,0 bis 10 Gew.-% Rhodium, 0,01 bis 20 Gew.-% Magnesium und 0,05 bis 15 Gew.-% Halogeniden.

Zur Durchführung des erfindungsgemässen Verfahrens werden Gasgemische, die ganz oder zu einem überwiegenden Teil aus Kohlenmonoxid und Wasserstoff bestehen und daneben gegebenenfalls noch andere Komponenten, wie Stickstoff, Argon, Kohlendioxid oder Methan enthalten können, über den Katalysator geleitet. Das molare Verhältnis von Kohlenmonoxid und Wasserstoff kann dabei in weiten Grenzen variiert werden. Bevorzugt sind Molverhältnisse zwischen 5:1 und 1:5 und besonders zwischen 3:1 und 1:3. Die Reaktionstemperaturen liegen im allgemeinen zwischen 175 und 375°C, vorzugsweise zwischen 200 und 350°C, und die Reaktionsdrücke zwischen 1 und 300 bar, vorzugsweise zwischen 20 und 200 bar.

Zweckmässig ist es, Temperatur und Druck so aufeinander abzustimmen, dass eine hohe Selektivität zu den sauerstoffhaltigen Verbindungen gewährleistet ist und die bei höheren Temperaturen begünstigte exotherme Bildung von Methan gering gehalten wird. Man bevorzugt deshalb hohe Drücke und möglichst niedrige Temperaturen. Der Umsatz an Kohlenmonoxid sollte dabei im allgemeinen unter 50% liegen, da höhere Umsätze leicht zu vermehrter Nebenproduktbildung führen können, wobei neben Methan, Kohlendioxid und gasförmigen Kohlenwasserstoffen auch höhermolekulare flüssige Kohlenwasserstoffe und sauerstoffhaltige Produkte auftreten können. Für die Verfahrensdurchführung ist die Gasphase bevorzugt. Hierzu können die herkömmlichen Festbettreaktoren verwendet werden, wobei es zur besseren Wärmeabführung vorteilhaft ist, die Katalysatorschichtdicke gering zu halten. Ferner sind auch Reaktoren mit bewegtem Katalysatorbett oder Wirbelbettreaktoren geeignet.

Man kann aber auch eine Umsetzung des Synthesegases in Gegenwart des festen und feinverteilten Katalysators, suspendiert in inerten Lösungsmitteln und/oder Reaktionsprodukten, durchführen.

Eine besonders bevorzugte Ausführungsform der Erfindung besteht darin, die Umsetzung in einer Kreisgasapparatur in der Gasphase durchzuführen, in der nach Abtrennung der kondensierbaren Reaktionsprodukte das nicht umgesetzte Gasgemisch wieder in den Reaktor zurückgeführt wird. Diese Verfahrensweise ist besonders wirtschaftlich und ermöglicht durch Verdünnung des Frischgases mit dem im Kreislauf zurückgeführten wasserstoffärmeren Restgas höhere Reaktionstemperaturen und damit höhere Raumzeitausbeuten bei unveränderten Selektivitäten. Als Kreisgasapparatur kommen dabei solche mit innerem oder äusseren Gasumlauf in Betracht.

Bei der Durchführung des erfindungsgemässen Verfahrens hat sich gezeigt, dass die Katalysatoren zwar eine hohe Anfangsaktivität und eine ausgezeichnete Selektivität der Kohlenmonoxidumsetzung zu den sauerstoffhaltigen $C_2$-Verbindungen aufweisen, dass aber bei längerem Einsatz der Katalysatoren, d.h. bei Betriebszeiten von mehr als etwa 500 Stunden, Aktivität und Selektivität der Katalysatoren allmählich abfallen können. Die Katalysatoren haben infolgedessen teilweise nur eine begrenzte Lebensdauer.

Es wurde nun gefunden, dass man diese beträchtlich verlängern kann, wenn man während der Synthesegasumsetzung der Reaktionszone zusammen mit den gasförmigen Reaktionskomponenten Magnesiumsalze oder -verbindungen, die unter den Reaktionsbedingungen verdampfbar sind, kontinuierlich oder diskontinuierlich zuführt.

Diese bevorzugte Ausführungsform des erfindungsgemässen Verfahrens bietet den Vorteil, dass die für die Umsetzung verwendeten Katalysatoren auf Basis Rhodium, Magnesium und Halogeniden in ihrer Aktivität und Selektivität auch nach mehr als 1000 Stunden noch nahezu unverändert sind.

Magnesiumsalze oder -verbindungen, die unter den Reaktionsbedingungen verdampfbar sind und dadurch gemeinsam mit einer oder mehreren der Reaktionskomponenten gasförmig in die Reaktionszone gebracht werden können, sind beispielsweise Magnesiumchlorid, -bromid, -jodid, -acetylacetonat, -ethylat, -isopropylat, Magnesiumaluminiumethylat und -isopropylat und die Magnesiumsalze aliphatischer Monocarbonsäuren mit 1–4 Kohlenstoffatomen im Molekül.

Vorzugsweise verwendet man Magnesiumchlorid oder Magnesiumacetat. Es lassen sich aber auch solche Magnesiumsalze oder -verbindungen verwenden, die durch Umsetzung mit Halogenwasserstoff in das Halogenid oder durch Umsetzung mit aliphatischen Monocarbonsäuren in die entsprechenden Carboxylate übergeführt werden könne, wie z.B. das Oxid, Hydroxid oder die Carbonate des Magnesiums.

Die flüchtigen Magnesiumsalze oder -verbindungen werden mit den gasförmigen Reaktionskomponenten in die Reaktionszone gebracht, wobei verschiedene Methoden angewendet werden können. So kann man die Magnesiumverbindungen in gelöster Form, beispielsweise als Lösung in Wasser, Ethanol oder Essigsäure, in den heissen Gasstrom vor der Katalysatorschicht einspritzen. Es besteht weiter die Möglichkeit, die Reaktionsgase vor Eintritt in die Reaktionszone ganz oder teilweise bei erhöhter Temperatur mit einer Lösung oder Schmelze der Magnesiumverbindung in Berührung zu bringen oder diese Gase über eine solche Lösung bzw. Schmelze zu leiten. Eine besonders bevorzugte Ausführungsform besteht darin, die Reaktionskomponenten teilweise oder insgesamt über die in fester Form vorgelegte flüchtige Magnesiumverbindung bei erhöhter Temperatur zu leiten und so die Magnesiumverbindung ohne Verwendung eines zusätzlichen Lösungsmittels zu verdampfen. Hierbei können die flüchtigen Magnesiumverbindungen auch auf einem inerten Trägermaterial, beispielsweise Kieselsäure, Aluminiumoxid oder Kohle, aufgebracht sein. Die zu verdampfende Magnesiumverbindung kann sich ausserhalb oder innerhalb des Reaktors befinden. Vorzugsweise ist sie so angeordnet, dass die erhitzten Reaktionskomponenten zuerst durch eine die Magnesiumverbindung enthaltende Zone und dann durch die den Katalysator enthaltende Zone des Reaktors strömen. Im Prinzip können diese beiden Zonen ineinander übergehen oder gegebenenfalls miteinander vermischt sein.

Die flüchtigen Magnesiumverbindungen können kontinuierlich oder diskontinuierlich in die Reaktionszone eingebracht werden. Bei der bevorzugten kontinuierlichen Zugabe der Magnesiumverbindung beträgt sie 0,01 bis 200 ppm, vorzugsweise 0,1 bis 50 ppm, bezogen auf das Gewicht des über den Katalysator geleiteten Gasstroms. Bei diskontinuierlichem Zusatz der Magnesiumverbindung können je nach der Dauer des Zusatzes gegebenenfalls auch grössere Mengen der Magnesiumverbindung dem Gasgemisch beigemischt werden. Die zugeführte Menge lässt sich über die Temperatur und das Volumen des über die Magnesiumverbindung geleiteten Gases regeln.

Der die flüchtige Magnesiumverbindung, Kohlenmonoxid und Wasserstoff enthaltende Gasstrom wird dann an dem Rhodium, Magnesium und Halogenid enthaltenden Katalysator umgesetzt.

Bei der besonders bevorzugten Ausführungsform in einer Kreisgasapparatur, in der nach Abtrennung der kondensierbaren Reaktionsprodukte das nicht umgesetzte Gasgemisch nach Zugabe von frischem Synthesegas wieder in den Reaktor zurückgeführt wird, kann die Magnesiumverbindung entweder dem Kreisgas, dem frischen Synthesegas oder dem Gemisch der beiden zugegeben werden.

Man kann die Lebensdauer der Katalysatoren aber auch auf eine andere Art als durch Zufuhr von Magnesiumverbindungen verlängern, nämlich dadurch, dass man während der Synthesegasumsetzung der Reaktionszone zusammen mit den gasförmigen Reaktionskomponenten Halogenwasserstoff oder flüchtige organische Halogenverbindungen, die keinen Schwefel oder Stickstoff im Molekül enthalten, und die unter den Reaktionsbedingungen Halogenwasserstoff abspalten, kontinuierlich oder diskontinuierlich zuführt.

Diese bevorzugte Ausführungsform des erfindungsgemässen Verfahrens bietet ebenso wie die Zufuhr von Magnesiumverbindungen den Vorteil, dass die für die Umsetzung verwendeten Katalysatoren auf Basis Rhodium, Magnesium und Halogeniden in ihrer Aktivität und Selektivität auch nach mehr als 1000 Stunden noch nahezu unverändert sind.

Als Halogenwasserstoffe können Chlorwasserstoff, Bromwasserstoff oder Jodwasserstoff bzw. deren Gemische eingesetzt oder durch Umsetzung von Halogenen mit Wasserstoff bzw. Synthesegas im Reaktionsraum erzeugt werden. Der bevorzugte Halogenwasserstoff ist Chlorwasserstoff.

Flüchtige organische Halogenverbindungen, die keinen Schwefel oder Stickstoff im Molekül enthalten und die unter den Reaktionsbedingungen Halogenwasserstoff abspalten, sind Alkyl-, Aryl- und Aralkylhalogenide mit einem oder mehreren Halogenatomen im Molekül, wie beispielsweise Dichlormethan, Tetrachlorkohlenstoff, Ethyljodid, 1,1-Dichlorethan, Allylchlorid, tert.-Butylchlorid oder Benzylchlorid, ferner gesättigte oder ungesättigte Halogencarbonsäuren, -aldehyde, - alkohole, -ketone oder -ether der aliphatischen, cycloaliphatischen oder aromatischen Reihe, zum Beispiel Mono-, Di- oder Trichloressigsäure, Jodessigsäure, Bromaceton, $\alpha,\beta$-Dichloridiethylether, 3-Chlor-crotonsäure (cis- oder trans) und p-Chlorbenzoesäure. Weiterhin sind auch Carbonsäurehalogenide geeignet, wie z.B. Acetylchlorid, -bromid und -jodid oder Mono-, Di- oder Trichloracetylchlorid, die unter Einwirkung des bei der Synthesegas-Umsetzung entstehenden Wassers sehr leicht Halogenwasserstoff abspalten können. Die bevorzugte Halogenverbindung ist Acetylchlorid.

Es ist nicht erforderlich, dass die Halogenwasserstoff-Abspaltung aus den flüchtigen organischen Halogenverbindungen quantitativ erfolgt, sondern es genügt bereits die Abspaltung geringer Mengen Halogenwasserstoff, um die Lebensdauer der Katalysatoren wesentlich zu verlängern.

Die Halogenwasserstoffe oder die Halogenwasserstoff abspaltenden organischen Verbindungen werden zusammen mit den gasförmigen Reaktionskomponenten in die Reaktionszone gebracht, wobei verschiedene Methoden angewendet werden können. So kann man die Halogenwasserstoffe oder die organischen Halogenverbindungen in gelöster Form, beispielsweise als Lösung in Wasser, Ethanol oder Essigsäure, in den heissen Gasstrom einbringen. Es besteht weiter die Möglichkeit, das gesamte Reaktionsgas – oder im Nebenstrom nur einen Teil dieses Gasstromes – vor Eintritt in die Reaktionszone über die feste oder flüssige organische Halogenverbindung zu leiten. Durch Wahl der Gasmenge, des Drucks und der Temperatur kann dabei entsprechend dem Partialdruck der eingesetzten Verbindung die gewünschte Menge zugegeben werden. Weiter können die organischen Halogenverbindungen auch in imprägnierter Form auf einem inerten Träger wie Kieselsäure, Aluminiumoxid oder Kohle aufgebracht sein, über den dann die Reaktionskomponenten, d.h. CO und $H_2$, geleitet werden.

Die Halogenwasserstoffe bzw. die flüchtigen organischen Halogenverbindungen können kontinuierlich oder diskontinuierlich in die Reaktionszone eingebracht werden. Bei der bevorzugten kontinuierlichen Zugabe beträgt ihre Konzentration 0,01 bis 500 ppm, vorzugsweise 0,1 bis 100 ppm, bezogen auf das Gewicht des über den Katalysator geleiteten Gasstroms. Bei diskontinuierlichem Zusatz können je nach der Dauer des Zusatzes gegebenenfalls auch grössere Mengen dem Gasgemisch beigemischt werden. Die zugesetzten Mengen sind dabei umgekehrt proportional zur Dauer des Zusatzes.

Der den Halogenwasserstoff bzw. die flüchtige organische Halogenverbindungen, Kohlenmonoxid und Wasserstoff enthaltende Gasstrom wird dann an dem Rhodium, Magnesium und Halogenid enthaltenden Katalysator umgesetzt.

Bei der besonders bevorzugten Ausführungsform in einer Kreisgasapparatur, in der nach Abtrennung der kondensierbaren Reaktionsprodukte das nicht umgesetzte Gasgemisch nach Zugabe von frischem Synthesegas wieder in den Reaktor zurückgeführt wird, kann der Halogenwasserstoff bzw. die organische Halogenverbindung entweder dem Kreisgas, dem frischen Synthesegas oder dem Gemisch der beiden zugegeben werden.

Die beiden beschriebenen Massnahmen zur Verlängerung der Lebensdauer der Katalysatoren – Zufuhr von Magnesiumverbindungen oder von Halogenwasserstoff bzw. organischer Halogenverbindung – können auch gemeinsam angewandt werden.

Die Erfindung soll durch die nachfolgenden Beispiele erläutert werden, wobei die Beispiele aber in keiner Weise einschränkend sein sollen.

Beispiele

Beispiele 1–7 und Vergleichsbeispiele 1 und 2:
a) Allgemeine Versuchsbeschreibung:

Die Apparatur besteht aus einem beheizten Reaktionsrohr von 1 m Länge und 16 mm innerem Durchmesser aus korrosionsbeständigem Stahl mit einer koaxial angebrachten Thermometerhülse von 6 mm äusserem Durchmesser, einem nachgeschalteten Kondensator, einer Vorlage für das Kondensat und einem Kompressor für die Rückführung eines Teils der nichtkondensierten Gase zum Reaktor (Kreisgas). Es werden jeweils 100 ml der unten beschriebenen Katalysatoren eingefüllt. Nach Spülen der Apparatur mit Stickstoff wird zunächst mit einem Synthesegas der Zusammensetzung 49 Vol.-% CO, 49 Vol.-% $H_2$, 1 Vol.-% $CO_2$, 1 Vol.-% $N_2$ (und geringe Mengen anderer Komponenten) ein Druck von 80 bar eingestellt und der Reaktor auf 300°C aufgeheizt. Während des Aufheizens und im weiteren Versuchsverlauf werden stündlich 500 NL Synthesegas der obigen Zusammensetzung über die Saugseite des Kompressors dem Kreisgas zugeführt und zusammen mit diesem über den Katalysator geleitet. Das den Reaktor verlassende Gasgemisch wird in dem (solegekühlten) Kondensator auf etwa +5°C abgekühlt und die kondensierten Anteile in der Vorlage aufgefangen. Das nicht kondensierte Restgas wird nach Vermischen mit frischem Synthesegas über den Kompressor wieder dem Reaktor zugeführt. Zur Aufrechterhaltung des Drucks und zur Ausschleusung von Nebenprodukten wird ein Teil des Restgases über ein Druckhalteventil als Abgas abgeleitet. Nach dieser Methode werden die nachstehend beschriebenen Katalysatoren geprüft. In der Tabelle sind die Laufzeit der Versuche, die Raumzeitausbeuten an sauerstoffhaltigen $C_2$-Produkten pro Liter Katalysator und Stunde bei Versuchsbeginn und -ende, die prozentuale Verteilung von Essigsäure, Acetaldehyd und Ethanol, bezogen auf den $C_2$-Anteil des Kondensats, sowie die Selektivität zu diesen Verbindungen (in Mol-% CO, bezogen auf umgesetztes CO) zusammengestellt. Geringe Mengen an entstandenem Ethylacetat bzw. Acetaldehyddiethylacetal werden in Essigsäure, Ethanol bzw. Acetaldehyd umgerechnet.

b) Katalysatorherstellung

Beispiel 1

40 g einer Kieselsäure mit 270 m²/g BET-Oberfläche, 1,22 ml/g Porenvolumen, 0,4 kg/l Schüttgewicht, einem pH von 7,4 (gemessen an einem Granulat von 2-3 mm Durchmesser), sowie einem Gehalt von 99,35 Gew.-% $SiO_2$ und 0,2 Gew.-% Na werden mit einer Lösung von 10,4 g Magnesiumchlorid (56%ig) in 45 ml Wasser getränkt, 2 Stunden bei 70°C und 2 Stunden bei 150°C getrocknet. Anschliessend wird 30 Minuten bei 900°C gesintert. Nach dem Erkalten wird mit einer Lösung von 5,3 g RhCl$_3 \cdot$ x $H_2O$ (37,8 Gew.-% Rh) in 45 ml Wasser getränkt und in gleicher Weise wie oben getrocknet. Der Katalysator wird in einem Strömungsrohr aus Glas durch 3-stündiges Überleiten von 30 Nl/h Wasserstoff bei 450-500°C unter Normaldruck reduziert. Er enthält nach der Reduktion 4,2 Gew.-% Rh, 3,1 Gew.-% Mg und 1,1 Gew.-% Cl. Die Raumzeitausbeute beträgt zu Versuchsbeginn 415 g sauerstoffhaltige $C_2$-Verbindungen pro Liter Katalysator und Stunde. Davon entfallen 52.3 Gew.-% auf Essigsäure, 43.6 Gew.-% auf Acetaldehyd und 4.1 Gew.-% auf Ethanol.

Vergleichsbeispiel 1 (mit Mg- und Halogenfreiem Katalysator)

5,6 g Rh $(NO_3)_3 \cdot 2$ $H_2O$ (31,3 Gew.-% Rh) werden in 45 ml Wasser gelöst und auf 40 g des in Beispiel 1 beschriebenen Kieselsäureträgers aufgezogen. Nach einer Ruhezeit von 2 Stunden wird der Katalysator bei 80°C und 260 mbar unter Überleiten von 1 Nl/h Stickstoff getrocknet. Der Katalysator wird, wie in Beispiel 1 angegeben, reduziert und enthält 4,2 Gew.-% Rh.

Vergleichsbeispiel 2 (mit Halogen-freiem Katalysator)

Eine Lösung von 14,5 g Mg $(NO_3)_2 \cdot 6$ $H_2O$ in 43 ml Wasser wird auf 40 g des in Beispiel 1 genannten Kieselsäureträgers aufgezogen. Der getränkte Träger wird bei 120°C getrocknet und anschliessend 30 Minuten bei 800°C gesintert. Nach dem Erkalten imprägniert man ihn mit einer Lösung von 5,9 g Rh $(NO_3)_3 \cdot 2$ $H_2O$ (31,3 Gew.-% Rh) in 45 ml Wasser, trocknet bei 80°C im Vakuum von 260 mbar unter 1 Nl/h Stickstoff und reduziert wie in Beispiel 1 beschrieben. Der Katalysator enthält nach der Reduktion 4,2 Gew.-% Rh, und 3,1 Gew.-% Mg.

Beispiel 2 (Reduktion im Reaktor)

Als Träger wird ein natürliches handelsübliches Magnesiumsilikat verwendet, das nach dem Waschen und Trocknen folgende Zusammensetzung hat:

65,5 Gew.-% $SiO_2$, 3,6 Gew.-% $Al_2O_3$, 0,5 Gew.-% $Fe_2O_3$ und 14,0 Gew.-% MgO. Das Schüttgewicht beträgt 537 g/l und das Porenvolumen 0,99 ml/g.

54 g dieses Trägers (100 ml) werden mit einer Lösung von 6,3 RhCl$_3 \cdot$ x $H_2O$ (37,8 Gew.-% Rh) in 49 ml Wasser getränkt und bei 150°C getrocknet. Der Katalysator wird unreduziert in den Reaktor gegeben, unter einem Stickstoffstrom von 30 Nl/h drucklos bis auf 425°C aufgeheizt und bei dieser Temperatur durch Überleiten von 60 Nl/h eines Gemisches von Kohlenmonoxid und Wasserstoff im Volumenverhältnis 1:1 reduziert. In Abweichung von der allgemeinen Versuchsbeschreibung wird nun der Katalysator auf 225°C abgekühlt, mit Synthesegas ein Druck von 80 bar eingestellt und der Katalysator auf die Reaktionstemperatur von 300°C aufgeheizt. Der Katalysator enthält nach der Reduktion 4,2 Gew.-% Rh, 7,8 Gew.-% Mg und 0,95 Gew.-% Cl.

Beispiel 3

Getrennt hergestellte Lösungen von 21 g MgBr$_2 \cdot 6$ $H_2O$ in 20 ml Wasser und 8,6 g RhBr$_3 \cdot 2$ $H_2O$ (27,2 Gew.-% Rh) in 14 ml Wasser werden vereinigt und sofort auf 40 g des in Beispiel 1 beschriebenen Kieselsäureträgers aufgezogen. Anschliessend wird bei 80°C und 260 mbar getrocknet und wie in Beispiel 1 reduziert.

**Beispiel 4**

Man löst getrennt voneinander 13,4 g $MgJ_2$ in 20 ml Wasser und 8,9 g $RhBr_3 \cdot 2\,H_2O$ (27,2 Gew.-% Rh) in 23 ml Wasser. Die beiden Lösungen werden auf 10°C abgekühlt, bei dieser Temperatur miteinander vereinigt und sofort auf 40 g des in Beispiel 1 beschriebenen Kieselsäureträgers aufgezogen. Anschliessend wird bei 90°C und einem Vakuum von 260 mbar unter Stickstoff getrocknet und wie in Beispiel 1 reduziert.

**Beispiel 5**

40 g Kieselsäureträger der in Beispiel 1 beschriebenen Zusammensetzung werden mit einer Lösung von 13,3 g $KMgCl_3 \cdot 6\,H_2O$ und 5,6 g $RhCl_3 \cdot$ x $H_2O$ (37,8 Gew.-% Rh) in 38 ml Wasser imprägniert, bei 120°C unter Nordmaldruck getrocknet und wie in Beispiel 1 reduziert.

**Beispiel 6**

Pyrogenes Titandioxid (hergestellt durch Flammenhydrolyse von $TiCl_4$) wird mit 2 Gew.-% Kaolin gemischt, mit Wasser angeteigt, getrocknet und auf eine Korngrösse von 0,1 – 1 mm zerkleinert. Anschliessend werden Tabletten von etwa 3 mm Durchmesser gepresst und diese bei 600°C gesintert.

80 g (entsprechend 100 ml) dieser Tabletten werden mit einer Lösung von 20 g $MgCl_2 \cdot H_2O$ (56 %ig) und 7,5 g $RhCl_3 \cdot$ x $H_2O$ (37,8 Gew.-% Rh) in 45 ml Wasser imprägniert, bei 150°C getrocknet und nach der in Beispiel 1 beschriebenen Methode reduziert. Der Katalysator enthält 3 Gew.-% Rh, 3 Gew.-% Mg und 1,03 Gew.-% Cl.

**Beispiel 7**

Feinst verteiltes Zirkondioxid wird wie in Beispiel 6 tablettiert. Auf 85 g (entsprechend 100 ml) dieser Tabletten wird eine Lösung von 22 g $MgCl_2 \cdot$ x $H_2O$ (56 % $MgCl_2$) und 16,5 g $RhCl_3 \cdot$ x $H_2O$ (37,8 % Rh) in 48 ml Wasser aufgezogen. Der Katalysator wird bei 90°C getrocknet und wie in Beispiel 1 reduziert.

**C) Versuchsergebnisse**

Die Ergebnisse sind in der folgenden Tabelle dargestellt.

**Tabelle**

Reaktionsbedingungen: 80 bar, 300°C, Einsatzgas 500 Nl/h $CO:H_2 = 1:1$, Katalysatorvolumen 0,1 l AcOH = Essigsäure, AcH = Acetaldehyd, EtOH = Ethanol

| Beispiel | Katalysatorzusammensetzung | | | Versuchs-Raumzeitausbeute laufzeit in sauerstoffhaltige $C_2$-Verb. in g/l·h | | | Zusammensetzung der $C_2$-Verbindungen in Gew.-% | | | Selektivität Mol % CO umgesetzt zu | |
|---|---|---|---|---|---|---|---|---|---|---|---|
| | Rh | Mg | Träger | Stunden | Versuchsbeginn | Versuchsende | AcOH | AcH | EtOH | $C_2$-Verb. | $CH_4$ |
| | [Gew.-%] | [Gew.-%] | | | | | | | | | |
| 1 | 4,2 | 3,1 | $SiO_2$ | 412 | 415 | 398 | 52,3 | 43,6 | 4,1 | 82,2 | 8,5 |
| Vergl. bsp.1 | 4,2 | — | $SiO_2$ | 194 | 61 | 42 | 45,5 | 18,7 | 35,8 | 51,1 | 35,6 |
| Vergl. bsp. 2 | 4,2 | 3,1 | $SiO_2$ | 194 | 256 | 238 | 58,6 | 28,8 | 12,6 | 66,7 | 18,9 |
| 2 | 4,2 | 8,4 | $MgSiO_3$ | 322 | 402 | 377 | 32,3 | 7,2 | 60,5 | 78,0 | 14,2 |
| 3 | 4,2 | 3,1 | $SiO_2$ | 186 | 395 | 362 | 54,7 | 36,0 | 9,3 | 75,2 | 19,4 |
| 4 | 4,6 | 2,2 | $SiO_2$ | 186 | 390 | 358 | 48,8 | 33,5 | 17,7 | 74,6 | 18,5 |
| 5 | 4,2 | 2,3 | $SiO_2$ | 212 | 398 | 370 | 38,2 | 19,5 | 42,3 | 80,5 | 11,2 |
| 6 | 3,0 | 3,0 | $TiO_2$ | 186 | 368 | 346 | 41,5 | 17,9 | 40,6 | 78,6 | 12,5 |
| 7 | 6,0 | 3,0 | $ZrO_2$ | 162 | 412 | 388 | 35,5 | 24,3 | 40,2 | 75,4 | 16,7 |

**Beispiel 8**

Als Träger wird ein natürlich vorkommendes, handelsübliches Magnesiumsilikat verwendet, das nach dem Waschen und Trocknen folgende Zusammensetzung hat:

65,5 Gew.-% $SiO_2$, 3,6 Gew.-% $Al_2O_3$, 0,5 Gew.-% $Fe_2O_3$ und 14,0 Gew.-% MgO. Das Schüttgewicht beträgt 537 g/l und das Porenvolumen 0,99 ml/g.

108 g dieses Trägers (200 ml) werden mit einer Lösung von 12,6 g Rh $Cl_3 \cdot$ x $H_2O$ (37,8 Gew.-% Rh) in 98 ml Wasser getränkt und bei 150°C getrocknet. Der Katalysator wird in einem Strömungsrohr aus Glas durch 3-stündiges Überleiten von 75 Nl/h Wasserstoff bei 375–425°C unter Nordmaldruck reduziert. Er enthält nach der Reduktion 4,2 Gew.-% Rh, 7,8 Gew.-% Mg und 1,05 Gew.-% Cl.

100 ml des reduzierten Katalysators werden in ein als Reaktor dienendes senkrecht stehendes Strömungsrohr aus korrosionsbeständigem Stahl von 16 mm innerem Durchmesser und 1 m Länge gegeben, das mit äusserer Salzbadheizung, Thermometer, Vorheizer, nachgeschaltetem Kühler, Vorlage für das Kondensat, Entspannungsventil und einem Kompressor für die Kreisgasführung eines Teils des Restgasmisches versehen ist. Der Vorheizer wird mit 100 ml eines Kieselsäureträgers gefüllt, der mit einer Lösung von 15 g Magnesiumacetat in 40 g Wasser getränkt und anschliessend getrocknet wurde.

Nach Spülen mit Stickstoff wird zunächst mit einem Synthesegas (49 Vol.% CO, 49 Vol.% H$_2$, 1 Vol.% CO$_2$, Spuren N$_2$) ein Druck von 120 bar eingestellt und der Katalysator auf 280°C aufgeheizt. Während des Aufheizens und im weiteren Versuchsverlauf werden stündlich 300 NL des Synthesegases über die Saugseite des Kompressors dem Kreisgas zugeführt und zusammen mit dem Kreisgas erst durch den auf 280°C beheizten Vorheizer und anschliessend durch den Reaktor geleitet. Das den Reaktor verlassende Gasgemisch wird in einem solegekühlten Kondensator auf ca. +5°C abgekühlt und die kondensierten Anteile werden in der Vorlage aufgefangen. Das nicht kondensierte Restgas wird nach dem Vermischen mit dem Frischgas über den Kompressor wieder dem Vorheizer und dem Reaktor zugeführt. Zur Konstanthaltung des Drucks und zur Ausschleusung von Nebenprodukten wird ein Teil des Restgases über ein Druckhalteventil als Abgas abgeleitet.

Es fallen stündlich 41 g sauerstoffhaltige C$_2$-Verbindungen (22 g Ethanol, 12 g Essigsäure, 7 g Acetaldehyd) in Form einer wässrigen Lösung an, entsprechend einer Raumzeitausbeute von 410 g pro Liter Katalysator und Stunde. Der CO-Umsatz beträgt durchschnittlich 31% von Einsatz und die Selektivität zu den sauerstoffhaltigen C$_2$-Produkten 82.3%, bezogen auf umgesetztes Kohlenmonoxid. Raumzeitausbeute, CO-Umsatz und Selektivität sind auch nach mehr als 1500 Stunden noch unverändert.

Geringe Mengen (etwa 2 Gew.-% bezogen auf die genannten C$_2$-Produkte) an entstandenem Ethylacetat bzw. Acetaldehyddiethylacetal wurden in Essigsäure, Ethanol bzw. Acetaldehyd umgerechnet und sind in den angegebenen Werten enthalten. Dies gilt auch für die folgenden Beispiele.

Vergleichsbeispiel 3

Man verfährt wie im Beispiel 8, füllt aber den Vorheizer mit reiner, nicht getränkter Kieselsäure. Unter sonst gleichen Versuchsbedingungen wie im Beispiel 8 und unter Verwendung von 100 ml Katalysator der dort genannten Zusammensetzung liegt die Raumzeitausbeute in den ersten 500 Stunden bei 400 g sauerstoffhaltigen C$_2$-Produkten pro Liter Katalysator und Stunde, nach insgesamt 740 Stunden bei 365 g/lh, nach 1000 Stunden bei 348 g/lh und nach 1680 Stunden nur noch bei 321 g/lh, bei gleicher prozentualer Zusammensetzung des Produktgemisches wie im Beispiel 8. Der CO-Umsatz fällt in der gleichen Zeit von 31% auf 29.9% und die Selektivität zu den sauerstoffhaltigen C$_2$-Produkten von über 80% auf 72.5%, bezogen auf umgesetztes Kohlenmonoxid.

Beispiel 9

100 g einer Kieselsäure mit 270 m²/g BET-Oberfläche, 1,27 ml/g Porenvolumen, 0,4 kg/l Schüttgewicht, einem pH von 7,4 (als Granulat von 2–3 mm Durchmesser), sowie einem Gehalt von 99.35 Gew.-% SiO$_2$ und 0,2 Gew.-% Na werden mit einer Lösung von 18,75 g Magnesiumchlorid (56 %ig) in 112 ml Wasser getränkt und dann 2 Stunden bei 70°C und 2 Stunden bei 150°C getrocknet. Anschliessend wird 30 Minuten bei 900°C gesintert. Nach dem Erkalten wird die Kieselsäure mit einer Lösung von 14,25 g RhCl$_3$ · x H$_2$O (37,8 Gew.-% Rh) in 112 ml Wasser getränkt und in gleicher Weise wie oben getrocknet. Der Katalysator wird in einem Strömungsrohr aus Glas durch 3-stündiges Überleiten von 75 Nl/h Wasserstoff bei 400–450°C unter Nordmaldruck reduziert. Er enthält nach der Reduktion 4,6 Gew.-% Rh, 2,3 Gew.-% Mg und 0,7 Gew.-% Cl.

100 ml des Katalysators werden in den in Beispiel 8 beschriebenen Reaktor gegeben, der jedoch im Unterschied zu Beispiel 8 nicht mit einem Kompressor versehen ist. Nach Spülen mit Stickstoff werden bei 120 bar und 280°C stündlich 235 Nl eines Gasgemisches, das 49 Vol.-% Kohlenmonoxid, 49 Vol.% Wasserstoff, 1 Vol.% Kohlendioxid und geringe Mengen Stickstoff enthält, über den Katalysator geleitet und zwar im einfachen Durchgang, d.h. nicht nach der Kreisgasfahrweise. In das heisse Gasgemisch werden in dem ebenfalls auf 280°C beheizten Vorheizer stündlich 10 ml einer wässrigen 0,07 gewichtsprozentigen Magnesiumacetat-Lösung eingespritzt.

Die Reaktionsgase werden nach Verlassen des Reaktors auf etwa –5°C abgekühlt und die nicht kondensierten Anteile entspannt. Es fallen als Kondensat stündlich 28 g Essigsäure, 8,5 g Acetaldehyd und 3,5 g Ethanol an. Der CO-Umsatz beträgt 35.1%, die Selektivität zu den sauerstoffhaltigen C$_2$-Produkten 81.0%, bezogen auf umgesetztes Kohlenmonoxid. Die Raumzeitausbeute, der CO-Umsatz und die Selektivität sind auch nach über 1000 Stunden noch unverändert.

Vergleichsbeispiel 4

Man verfährt wie in Beispiel 9, gibt jedoch anstelle der wässrigen Magnesiumacetatlösung stündlich 10 ml destilliertes Wasser in den Vorheizer. Die Raumzeitausbeute beträgt nach 200 Stunden 395 g sauerstoffhaltige C$_2$-Produkte, nach 600 Stunden noch 360 g und nach 1000 Stunden noch 290 g, jeweils pro Liter Katalysator und Stunde. Die prozentuale Zusammensetzung der C$_2$-Produkte ist die gleiche wie in Beispiel 9. Der CO-Umsatz geht in dieser Zeit von 35% auf 32,5% und die Selektivität zu den sauerstoffhaltigen C$_2$-Produkten von 80.7% auf 63.5% zurück.

Beispiel 10

100 g einer Kieselsäure mit 270 m²/g BET-Oberfläche, 1,27 ml/g Porenvolumen, 0,4 kg/l Schüttgewicht, einem pH von 7,4 (als Granulat von 2–3 mm Durchmesser), sowie einem Gehalt von 99.35 Gew.-% SiO$_2$ und 0,2 Gew.-% Na werden mit einer Lösung von 14,7 g Magnesiumchlorid (56 %ig) in 112 ml Wasser getränkt und dann 2 Stunden bei 150°C getrocknet. Anschliessend wird 30 Minuten bei 800°C gesintert. Nach dem Erkalten wird die Kieselsäure mit einer Lösung von 14,0 g RhCl$_3$ · xH$_2$O (38,0 Gew.-% Rh) in 112 ml Wasser getränkt und in gleicher Weise wie oben getrocknet. Der Katalysator wird in einem Strömungsrohr aus Glas durch 3-stündiges Überleiten von 75 Nl/h

Wasserstoff bei 400–450°C unter Nordmaldruck reduziert. Er enthält nach der Reduktion 4,5 Gew.-% Rh, 1,8 Gew.-% Mg und 0,6 Gew.-% Cl.

100 ml des reduzierten Katalysators werden in ein als Reaktor dienendes senkrecht stehendes Strömungsrohr aus korrosionsbeständigem Stahl von 16 mm innerem Durchmesser und 1 m Länge gegeben, das mit äusserer Salzbadheizung, Thermometer, Vorheizer, nachgeschaltetem Kühler, Vorlage für das Kondensat und Entspannungsventil versehen ist.

Nach Spülen mit Stickstoff werden bei 100 bar und 290°C stündlich 250 Nl eines Gasgemisches, das 49 Vol.% Kohlenmonoxid, 49 Vol.% Wasserstoff, 1 Vol.% Kohlendioxid und geringe Mengen Stickstoff enthält, über den Katalysator geleitet. In das heisse Gasgemisch werden in dem ebenfalls auf 290°C beheizten Vorheizer stündlich 10 ml einer wässrigen 0,1 gewichtsprozentigen Salzsäure eingespritzt.

Die Reaktionsgase werden nach Verlassen des Reaktors auf etwa +5°C abgekühlt und die nicht kondensierten Anteile entspannt. Es fallen als Kondensat stündlich 27 g Essigsäure, 6,5 g Acetaldehyd und 2,5 g Ethanol an, entsprechend einer Raumzeitausbeute von 360 g/l·h. Der CO-Umsatz beträgt 28.9%, die Selektivität zu den sauerstoffhaltigen $C_2$-Produkten 82,5%, bezogen auf umgesetztes Kohlenmonoxid. Die Raumzeitausbeute, der CO-Umsatz und die Selektivität sind auch nach 1000 Stunden noch unverändert.

Geringe Mengen (etwa 3 Gew.-%, bezogen auf die genannten $C_2$-Produkte) an gleichzeitig entstandenem Ethylacetat bzw. Acetaldehyddiethylacetal wurden in Essigsäure, Ethanol bzw. Acetaldehyd umgerechnet und sind in den angegebenen Werten enthalten. Dies gilt auch für die folgenden Beispiele.

Vergleichsbeispiel 5

Man verfährt wie in Beispiel 10, gibt jedoch anstelle der verdünnten Salzsäure stündlich 10 ml destilliertes Wasser in den Vorheizer. Die Raumzeitausbeute beträgt nach 240 Stunden 345 g sauerstoffhaltige $C_2$-Produkte, nach 600 Stunden noch 315 und nach 1200 Stunden noch 240 g, jeweils pro Liter Katalysator und Stunde. Die prozentuale Zusammensetzung der $C_2$-Produkte ist die gleiche wie in Beispiel 10. Der CO-Umsatz geht in dieser Zeit von 28,2% auf 24,8% und die Selektivität zu den sauerstoffhaltigen $C_2$-Produkten von 81,0% auf 64,0% zurück.

Beispiel 11

a) Als Träger wird ein natürlich vorkommendes, handelsübliches Magnesiumsilikat verwendet, das nach dem Waschen und Trocknen folgende Zusammensetzung hat:

65,5 Gew.-% $SiO_2$, 3,6 Gew.-% $Al_2O_3$, 0,5 Gew.-% $Fe_2O_3$ und 14,0 Gew.-% MgO. Das Schüttgewicht beträgt 537 g/l und das Porenvolumen 0,99 ml/g. 108 g dieses Trägers (200 ml) werden mit einer Lösung von 12,6 g $RhCl_3 \cdot x\ H_2O$ (37,8 Gew.-% Rh) in 98 ml Wasser getränkt und bei 150°C getrocknet. Der Katalysator wird in einem Strömungsrohr aus Glas durch 3-stündiges Überleiten von 75 Nl/h Wasserstoff bei 375–425°C unter Normaldruck reduziert. Er enthält nach der Reduktion 4,1 Gew.-% Rh, 7,5 Gew.-% Mg und 1,12 Gew.-% Cl.

100 ml des Katalysators werden in den in Beispiel 10 beschriebenen Reaktor gegeben, der jedoch im Unterschied zu Beispiel 10 zusätzlich mit einem Kompressor für die Kreisgasführung eines Teils des Restgasgemisches versehen ist.

Nach Spülen mit Stickstoff wird zunächst mit einem Synthesegas (49 Vol.% CO, 49 Vol.% $H_2$, 1 Vol.% $CO_2$, Spuren $N_2$) ein Druck von 100 bar eingestellt und der Katalysator auf 290°C aufgeheizt. Während des Aufheizens und im weiteren Versuchsverlauf werden stündlich 300 Nl des Synthesegases über die Saugseite des Kompressors dem Kreisgas zugeführt und zusammen mit dem Kreisgas erst durch den auf 290°C beheizten Vorheizer und anschliessend durch den Reaktor geleitet. In den Vorheizer werden stündlich 10 ml einer 0,1 gewichtsprozentigen Lösung von 1,1-Dichlorethan in Methanol eindosiert. Das den Reaktor verlassende Gasgemisch wird in einem solegekühlten Kondensator auf ca. +5°C abgekühlt und die kondensierten Anteile in der Vorlage aufgefangen. Das nicht kondensierte Restgas wird nach dem Vermischen mit dem Frischgas über den Kompressor wieder dem Vorheizer und dem Reaktor zugeführt. Zur Konstanthaltung des Drucks und zur Ausschleusung von Nebenprodukten wird ein Teil des Restgases über ein Druckhalteventil als Abgas abgeleitet.

Es fallen stündlich 38 g sauerstoffhaltige $C_2$-Verbindungen (21 g Ethanol, 10 g Essigsäure, 7 g Acetaldehyd) in Form einer wässrigen Lösung an, entsprechend einer Raumzeitausbeute von 380 g pro Liter Katalysator und Stunde. Der CO-Umsatz beträgt durchschnittlich 28.6% vom Einsatz und die Selektivität zu den sauerstoffhaltigen $C_2$-Produkten 83,3%, bezogen auf umgesetztes Kohlenmonoxid. Raumzeitausbeute, CO-Umsatz und Selektivität sind auch nach mehr als 1800 Stunden noch unverändert.

Anstelle der 0,1 gewichtsprozentigen Lösung von 1,1-Dichlorethan in Methanol wurde in drei folgenden, ansonsten identischen Versuchen eine gleichkonzentrierte Lösung von

b) Monochloressigsäure in Methanol,
c) von Benzylchlorid in Methanol und
d) von Acetylchlorid in Diethylether verwendet.

Die Ergebnisse waren jeweils dieselben wie bei a).

Vergleichsbeispiel 6

Man verfährt wie im Beispiel 11, gibt aber in den Vorheizer stündlich 10 ml reines Methanol. Unter sonst gleichen Versuchsbedingungen wie im Beispiel 11 und unter Verwendung von 100 ml Katalysator der dort genannten Zusammensetzung liegt die Raumzeitausbeute in den ersten 600 Stunden bei 360 g sauerstoffhaltigen $C_2$-Produkten pro Liter Katalysator und Stunde, nach 1000 Stunden bei 315 g/l·h und nach 1500 Stunden nur noch bei 280 g/l·h, bei gleicher prozentualer Zusammensetzung des Produktgemisches wie im Beispiel 11. Der CO-Umsatz fällt in der gleichen Zeit von 27,9% auf 24,1% und die Selekti-

vität zu den sauerstoffhaltigen $C_2$-Produkten von 81,2% auf 72,6%, bezogen auf umgesetztes Kohlenmonoxid.

## Patentansprüche

1. Verfahren zur Herstellung von Essigsäure, Ethanol, Acetaldehyd und gegebenenfalls ihren Folgeprodukten durch Umsetzung von Kohlenmonoxid mit Wasserstoff an Rhodiummetall enthaltenden Trägerkatalysatoren, dadurch gekennzeichnet, dass die Katalysatoren zusätzlich einerseits Halogenidionen und andererseits Magnesiumsalze und/oder -komplexverbindungen und/oder Verbindungen des Magnesiums mit Oxiden von Elementen der III. bis VI. Gruppe des periodischen Systems enthalten.

2. Verfahren nach Anspruch 1, dadurch gekennzeichnet, dass man als Träger Kieselsäure oder Silikate verwendet.

3. Verfahren nach Anspruch 1, dadurch gekennzeichnet, dass man als Träger Magnesiumsilikat verwendet.

4. Verfahren nach einem der Ansprüche 1 bis 3, dadurch gekennzeichnet, dass man als Halogenidionen Chloride verwendet.

5. Verfahren nach einem der Ansprüche 1 bis 4, dadurch gekennzeichnet, dass man die Katalysatoren bei 350–550°C reduziert.

6. Verfahren nach einem der Ansprüche 1 bis 5, dadurch gekennzeichnet, dass man der Reaktionszone Magnesiumsalze oder -verbindungen, die unter den Reaktionsbedingungen verdampfbar sind, während der Umsetzung zusammen mit den gasförmigen Einsatzkomponenten kontinuierlich oder diskontinuierlich zuführt.

7. Verfahren nach Anspruch 6, dadurch gekennzeichnet, dass man Magnesiumacetat oder Magnesiumchlorid verwendet.

8. Verfahren nach einem der Ansprüche 1 bis 7, dadurch gekennzeichnet, dass man der Reaktionszone Halogenwasserstoff oder flüchtige organische Halogenverbindungen, die keinen Schwefel oder Stickstoff im Molekül enthalten und die unter den Reaktionsbedingungen Halogenwasserstoff abspalten, während der Umsetzung zusammen mit den gasförmigen Einsatzkomponenten kontinuierlich oder diskontinuierlich zuführt.

9. Verfahren nach Anspruch 8, dadurch gekennzeichnet, dass man als Halogenwasserstoff Chlorwasserstoff verwendet.

10. Verfahren nach Anspruch 8, dadurch gekennzeichnet, dass man als organische Halogenverbindung Acetylchlorid verwendet.

## Claims

1. A process for the manufacture of acetic acid, ethanol, acetaldehyde and possibly the secondary products there of by reaction of carbon monoxide with hydrogen in the presence of rhodium metal-containing carrier catalysts, characterized in that the catalysts contain in addition on the one hand halide ions and on the other hand magnesium salts and/or magnesium complex compounds and/or compounds of magnesium with oxides of elements of the IIIrd to VIth group of the Periodic Table.

2. The process as claimed in claim 1, wherein silicic acid or silicates are used as carrier.

3. The process as claimed in claim 1, wherein magnesium silicate is used as carrier.

4. The process as claimed in one of the claims 1 to 3, wherein chlorides are used as halide ions.

5. The process as claimed in one of the claims 1 to 4, wherein the catalysts are reduced at 350 to 550°C.

6. The process as claimed in one of the claims 1 to 5, which comprises feeding continuously or discontinuously to the reaction zone during the reaction and together with the gaseous starting components magnesium salts or magnesium compounds vaporizable under the reaction conditions.

7. The process as claimed in claim 6, wherein magnesium acetate or magnesium chloride is used.

8. The process as claimed in one of the claims 1 to 7, which comprises feeding continuously or discontinuously to the reaction zone during the reaction and together with the gaseous starting components a hydrogen halide or a volatile organic halogen compound not containing any sulfur or nitrogen in the molecule and splitting off hydrogen halide under the reaction conditions.

9. The process as claimed in claim 8, wherein hydrogen chloride is used as hydrogen halide.

10. The process as claimed in claim 8, wherein acetyl chloride is used as organic halogen compound.

## Revendications

1. Procédé de préparation d'acide acétique, d'éthanol, d'acétaldéhyde et éventuellement de leurs produits successeurs, par réaction du monoxyde de carbone avec l'hydrogène en présence de catalyseurs sur support renfermant du rhodium métallique, procédé caractérisé en ce que les catalyseurs contiennent en outre, d'une part, des ions halogénures et, d'autre part, des sels et/ou complexes du magnésium et/ou des composés du magnésium avec des oxydes d'éléments des groupes III à VI de la Classification Périodique.

2. Procédé selon la revendication 1, caractérisé en ce qu'on utilise, comme supports, la silice ou des silicates.

3. Procédé selon la revendication 1, caractérisé en ce qu'on utilise un silicate de magnésium comme support.

4. Procédé selon l'une quelconque des revendications 1 à 3, caractérisé en ce qu'on utilise des chlorures comme ions halogénures.

5. Procédé selon l'une quelconque des revendications 1 à 4, caractérisé en ce qu'on réduit les catalyseurs à une température comprise entre 350 et 550°C.

6. Procédé selon l'une quelconque des revendications 1 à 5, caractérisé en ce qu'on introduit dans la zone réactionnelle, en continu ou en discontinu, au cours de la réaction, en même temps que les composantes gazeuses à mettre en jeu, des sels ou composés du magnésium susceptibles d'être évaporés dans les conditions de la réaction.

7. Procédé selon la revendication 6, caractérisé en ce qu'on utilise de l'acétate de magnésium ou de chlorure de magnésium.

8. Procédé selon l'une quelconque des revendications 1 à 7, caractérisé en ce qu'on introduit dans la zone réactionnelle, en continu ou en discontinu, au cours de la réaction, en même temps que les composantes gazeuses à mettre en jeu, un halogénure d'hydrogène ou des composés organiques halogénés volatils ne contenant ni soufre ni azote dans leur molécule et capables de libérer un halogénure d'hydrogène dans les conditions de la réaction.

9. Procédé selon la revendication 8, caractérisé en ce qu'on utilise le chlorure d'hydrogène comme halogénure d'hydrogène.

10. Procédé selon la revendication 8, caractérisé en ce qu'on utilise le chlorure d'acétyle comme composé organique halogéné.